# EUROPÄISCHE PATENTANMELDUNG

(11) **EP 0 525 370 A1**
(43) Veröffentlichungstag der Anmeldung: **03.02.1993**
(21) Anmeldenummer: 92110379.2
(22) Anmeldetag: 19.06.1992
(51) Int. Cl.: A61B 17/22

(54) **Schallwellenbehandlungsgerät**

(30) Priorität: 22.07.1991 DE 4124259
(71) Anmelder: Richard Wolf GmbH, D-75438 Knittlingen (DE)
(72) Erfinder: Bauer, Edgar, W-7527 Kraichtal 3 (DE); Krauss, Werner, W-7134 Knittlingen (DE); Burkhardt, Michael, W-7130 Mühlacker (DE); Fladl, Joachim, W-7519 Oberderdingen 3 (DE)
(74) Vertreter: Vollmann, Heiko, Dipl.-Ing.

(57) **Zusammenfassung**

Das Schallwellenbehandlungsgerät weist einen Schallerzeuger (4) und eine mit Koppelflüssigkeit füllbare Vorlaufstrecke (7) mit einer Membran (10) zwischen Schallerzeuger (4) und Patient (5) auf, wobei die Vorlaufstrecke (7) durch die Membran (10) in einen ersten abgeschlossenen und mit Koppelflüssigkeit füllbaren Raum (8) sowie einen zweiten offenen und durch den Patientenkörper (5) abschließbaren, ebenfalls mit Koppelflüssigkeit füllbaren Raum (9) getrennt ist. Der zweite Raum (9) ist volumenmäßig erheblich kleiner gestaltet als der erste, so daß zwar eine vorteilhafte unmittelbare Ankopplung der Vorlaufstrecke (7) unter weitestgehendem Ausschluß von Gasblasen an den Patientenkörper (5) erfolgt, jedoch nach der Behandlung nur ein geringes Volumen an Koppelflüssigkeit aus Gründen der Hygiene ausgetauscht werden muß.

## Beschreibung

Die Erfindung betrifft ein Schallwellenbehandlungsgerät, insbesondere eine Lithotripsieeinrichtung, mit einem Schallwellenerzeuger und einer mit Koppelflüssigkeit füllbaren Vorlaufstrecke mit einer Membran zwischen Schallerzeuger und Patient.

Um Schallwellen möglichst verlustfrei von einem Schallerzeuger in den menschlichen Körper einzukoppeln ist es erforderlich, das den Menschen sonst umgebende Gasgemisch zumindest in diesem Bereich zwischen Schallerzeuger und Körper durch eine Flüssigkeit zu ersetzen. Bei Lithotripsieeinrichtungen bezeichnet man diesen flüssigkeitsgefüllten Bereich zwischen Schallerzeuger und Körper als Vorlaufstrecke.

Bei Lithotripsieeinrichtungen der oben genannten Art ist diese Vorlaufstrecke zum Patienten hin durch eine elastische Membran abgeschlossen, um so den direkten Kontakt zwischen der Koppelflüssigkeit und dem Patientenkörper zu vermeiden. Um eine großflächige und gute Ankoppelung dieser Membran an den Körper zu gewährleisten, wird ein Koppelgel verwendet, ähnlich wie dies auch bei diagnostischen Schallwellengeräten zur Ankoppelung erfolgt.

Geräte der vorbeschriebenen Art sind beispielsweise aus DE 33 19 871 C2 und DE 34 25 992 C2 bekannt.

Zwar kann bei diesen Geräten durch Entgasung die Vorlaufstrecke zwischen Schallwellenerzeuger und Membran weitestgehend von unerwünschten Gaseinschlüssen freigehalten werden, doch ist dies im Bereich zwischen Membran und Patientenkörper trotz Koppelmediums nicht immer in der erforderlichen Weise möglich. Insbesondere durch Hautfalten und Körperhaare kann es in diesem Bereich zu Gasblaseneinschlüssen kommen, die die Schallwelleneinkoppelung in den Körper und somit die Therapie behindern. Ein Teil der Schallwellenenergie wird dann an diesen Gasblasen umgesetzt, was zu Hautrötungen führen kann.

Die vorstehend beschriebenen Nachteile durch unvollständige Ankoppelung des Schallwellenerzeugers an den Patienten können dadurch behoben werden, daß die Vorlaufstrecke unmittelbar an den Patientenkörper gelegt wird, also ohne Zwischenschaltung einer Membran. Ein derartiges Gerät ist beispielsweise aus DE 35 32 678 C2 bekannt. Ein erheblicher Nachteil dieser direkten Ankoppelung der Vorlaufstrecke an den Patientenkörper ist jedoch der direkte Kontakt der gesamten Koppelflüssigkeit mit dem Patientenkörper. Aus hygienischen Gründen ist es in der Praxis erforderlich, nach jedem Patientenwechsel die gesamte in der Vorlaufstrecke befindliche Koppelflüssigkeit auszutauschen. Da es sich hierbei um nicht unerhebliche Mengen temperierter Flüssigkeit handelt, geht hiermit nicht nur ein erheblicher Energie- und Flüssigkeitsverbrauch einher, sondern es sind auch erhebliche apparative Vorkehrungen zu treffen, damit das Befüllen, Temperieren und Entleeren der Vorlaufstrecke in annehmbar kurzer Zeit erfolgen kann. Alternativ könnte die Koppelflüssigkeit zwar nach jeder Behandlung aufbereitet werden, der apparative Aufwand hierzu ist jedoch noch größer.

Schließlich ist die in DE 35 32 678 C2 anhand von Figur 4 alternativ beschriebene Ausführung, bei der ein zum Patientenkörper hin offener Beutel in die nach oben offene Vorlaufstrecke eingehängt wird, ebenfalls von Nachteil. Zum einen hat es sich in der Praxis als äußerst schwierig erwiesen, diesen Beutel so an den Patientenkörper anzuschließen, daß keine Gasblasen eingeschlossen werden, zum anderen ist auch dort die Koppelflüssigkeit nach oben hin frei zugänglich, so daß eine Kontamination nicht mit genügender Sicherheit ausgeschlossen werden kann und die Koppelflüssigkeit zumindest in kürzeren Abständen zu erneuern bzw. zu regenerieren ist.

Ausgehend von dem einleitend erörterten Stand der Technik liegt der Erfindung die Aufgabe zugrunde, ein Schallwellenbehandlungsgerät so auszubilden, daß mit einfachen konstruktiven Mitteln eine möglichst optimale Ankoppelung an den Patientenkörper gewährleistet ist, wobei insbesondere die vorgenannten Nachteile vermieden werden sollen.

Diese Aufgabe wird erfindungsgemäß dadurch gelöst, daß die Vorlaufstrecke konstruktiv unterteilt wird, und zwar in einen ersten nach außen hin abgeschlossenen Raum zwischen Wandler und Membran sowie in einen zweiten offenen und durch den Patientenkörper abschließbaren Raum zwischen Membran und Patientenkörper. Die Erfindung verbindet also die Vorteile der durch Membran abgeschlossenen Vorlaufstrecke mit denen der unmittelbar angekoppelten Vorlaufstrecke. Denn es kann auf diese Weise der volumenmäßig größere Teil der Vorlaufstrecke in den ersten abgeschlossenen Raum verlegt werden. In diesem Bereich ist eine Kontamination der Koppelflüssigkeit ausgeschlossen, so daß ein Austausch oder eine Regeneration vollständig entfallen kann. Lediglich der volumenmäßig kleine Raum zwischen Membran und Patientenkörper ist als offene Vorlaufstrecke ausgebildet, in diesem Bereich muß die Koppelflüssigkeit beim Patientenwechsel ausgetauscht bzw. regeneriert werden. Es versteht sich, daß dies bei einem verhältnismäßig kleinen Volumen völlig unproblematisch ist. Es kann jedoch auf diese Weise die bevorzugte unmittelbare Ankoppelung der Vorlaufstrecke an den Patientenkörper erreicht werden, die ggf. durch Entgasung der Koppelflüssigkeit oder weiter unten noch beschriebene Maßnahmen optimiert werden kann.

Vorteilhafte Ausgestaltungen der Erfindung sind in den Unteransprüchen sowie in der Beschreibung aufgeführt.

Das erfindungsgemäße Schallwellenbehandlungsgerät ist hinsichtlich der Volumen der beiden Räume der Vorlaufstrecke vorteilhaft so ausgebildet, daß der zweite Raum ein möglichst kleines Volumen aufweist, so daß die Menge der auszutauschenden Koppelflüssigkeit möglichst gering ist. Das Volumen muß jedoch so groß sein, daß eine sichere Ankoppelung von Patienten unterschiedlichster Größe gewährleistet ist. Das Volumen des ersten abgeschlossenen Raumes kann daher ein Vielfaches des zweiten durch den Patientenkörper abschließbaren Raumes aufweisen.

Um einen dichten Abschluß des zweiten Raumes durch den Patientenkörper zu gewährleisten ist es von Vorteil, wenn der zweite Raum in seinem oberen seitlichen Bereich durch ein ringförmiges elastisches Kissen begrenzt wird.

Zum Befüllen und Entleeren des zweiten Raumes mit Koppelflüssigkeit ist es zweckmäßig, mindestens einen Kanal vorzusehen. Schon aus hygienischen Gründen ist es jedoch vorteilhaft, mindestens zwei solcher Kanäle in der Wandung des zweiten Raums vorzusehen, und zwar mindestens einen für die Zufuhr und mindestens einen weiteren für die Abfuhr der Koppelflüssigkeit. Bei der Anordnung mehrerer Kanäle sollten diese vorzugsweise in Gruppen und voneinander entfernt angeordnet sein, um eine Kontamination der zugeführten Koppelflüssigkeit mit Resten der bereits abgeführten Koppelflüssigkeit sicher zu vermeiden.

Zum Zwecke der Entgasung sowie zum Zwekke der Regeneration der im zweiten Raum befindlichen Koppelflüssigkeit ist es von Vorteil, wenn der zweite Raum so ausgebildet ist, daß die Koppelflüssigkeit diesen durchströmen kann. Es kann dann beispielsweise auch eine Temperierung der Koppelflüssigkeit erfolgen.

Konstruktiv von besonderem Vorteil ist es, wenn der zweite Raum umfangseitig durch einen Bajonettring abgeschlossen wird, der zum einen die Membran am Gerät, insbesondere an der Patientenauflage festlegt, zum anderen das elastische Kissen zum dichten Anschluß an den Patientenkörper aufnimmt und schließlich Kanäle zur Zu- und Abfuhr der Koppelflüssigkeit aufweist. In seinem unteren Bereich kann ein solcher Bajonettring ähnlich wie der der europäischen Patentanmeldung Nr. 90 118 764.1 beschriebene ausgebildet sein, was den besonderen Vorteil hat, daß derartige bereits vorhandene Schallwellenbehandlungsgeräte auch nachträglich mit der Erfindung ausgerüstet werden können. Im übrigen kann das erfindungsgemäße Gerät, sofern dies im Einzelfall zweckmäßig sein sollte, durch Austausch dieses Bajonettrings in ein herkömmliches Gerät umgebaut werden. Im Zusammenhang mit der vorliegenden Erfindung sei ausdrücklich auf die vorgenannte europäische Patentanmeldung Nr. 90 118 764.1 hingewiesen, die dort beschriebenen Merkmale können großteils in Kombination mit der vorliegenden Erfindung vorteilhaft eingesetzt werden.

Ein weiterer Vorteil dieser bajonettartigen Ausbildung der Wandung des zweiten Raumes ist der, daß der Bajonettring unabhängig von der Membran und umgekehrt ausgetauscht werden kann.

Zwar ist die vorstehende Erfindung anhand einer Lithotripsieeinrichtung beschrieben, sie kann jedoch auch für andere Therapiegeräte, beispielsweise bei der Hyperthermie oder auch bei diagnostischen Geräten angewandt werden. Die Erfindung ist nachfolgend anhand eines Ausführungsbeispiels näher beschrieben, das in den Figuren dargestellt ist. Es zeigen:
Figur 1 einen Teil einer Lithotripsieeinrichtung im Schnitt,
Figur 2 eine Draufsicht auf den Bajonettring in Figur 1 und
Figur 3 in vergrößerter und teilweise geschnittener Darstellung einen Teil des Bajonettrings nach Figur 2.

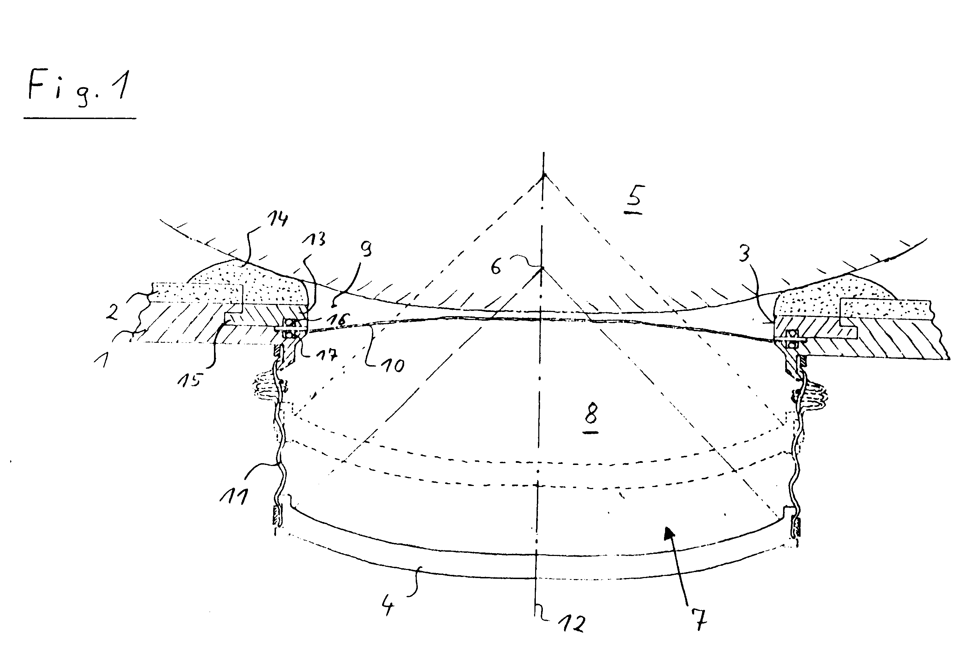

Die Lithotripsieeinrichtung nach Figur 1 weist eine Patientenauflage 1 auf, die nach oben mit einem Polster 2 abgedeckt ist. Die Patientenauflage 1 weist eine als Fenster bezeichnete Ausnehmung 3 auf, durch welche die Schallwellen eines Stoßwellenerzeugers 4 zu dem auf der Patientenauflage 1 befindlichen Patientenkörper 5 gelangen.

Bei dem dargestellten Stoßwellenerzeuger 4 handelt es sich um eine fokussierende Wandlerkalotte, auf der eine Vielzahl von piezoelektrischen Elementen nebeneinander angeordnet sind, die ein elektrisches Signal in Schallwellen wandeln. Der Fokus des Stoßwellenerzeugers 4 ist mit 6 bezeichnet. Wie sich aus der Zeichnung ergibt, ist der Stoßwellenerzeuger 4 mindestens in Richtung senkrecht zur Patientenauflage 1 verschiebbar gelagert.

Der Bereich zwischen dem Stoßwellenerzeuger 4 und dem Patientenkörper 5 - soweit er innerhalb des Fensters 3 befindlich ist - bildet eine Vorlaufstrecke 7, die mit einer Koppelflüssigkeit füllbar ist. Die Vorlaufstrecke 7 besteht aus einem ersten abgeschlossenen Raum 8 sowie einem zweiten abschließbaren Raum 9, die voneinander durch eine elastische Membran 10 getrennt sind.

Der erste Raum 8 wird umgrenzt durch den Stoßwellenerzeuger 4, die nach oben abschließende Membran 10 sowie einen seitlichen Balg 11, der einerseits an der Unterseite der Patientenauflage 1 das Fenster 3 umgebend und andererseits an der Außenseite des Stoßwellenerzeugers 4 dicht festgelegt ist. Dieser erste Raum 8 ist mit Koppelflüssigkeit gefüllt, wobei nicht dargestellte Ausgleichsgefäße vorgesehen sind, die dafür sorgen, daß beim Verschieben des Stoßwellenerzeugers 4 entlang der Achse 12 - also beim Strecken bzw. Zusammenziehen des Balgs 11 - das im ersten Raum 8 veränderte Volumen ausgeglichen wird. Es ist weiterhin eine nicht dargestellte Einrichtung zur Entfernung von Luftblasen aus diesem Raum 8 vorgesehen. Derartige Einrichtungen zum Ausgleich des Volumens und zur Abscheidung von Luftblasen sind an sich bekannt und daher nicht weiter beschrieben.

Der zweite Raum 9 wird nach unten hin durch die Membran 10 und seitlich durch einen Bajonettring 13, der nach oben durch ein ringförmiges elastisches Kissen 14 abgedeckt ist, das auch den angrenzenden Randbereich der Patientenauflage 1 übergreift, abgeschlossen. Dieser Raum 9 ist nach oben hin zunächst offen und bei der Behandlung durch den das Fenster 3 abdeckenden Teil des Patientenkörpers 5 abschließbar. Über das elastische Kissen 14 erfolgt ein dichter Anschluß der Vorlaufstrecke 7 an den Patientenkörper 5. Dieser zweite Raum 9 ist während der Behandlung ebenfalls mit Koppelflüssigkeit gefüllt, so daß sich eine durchgehende, lediglich durch die Membran 10 getrennte, unmittelbar an den Patientenkörper 5 angekoppelte Vorlaufstrecke 7 ergibt.

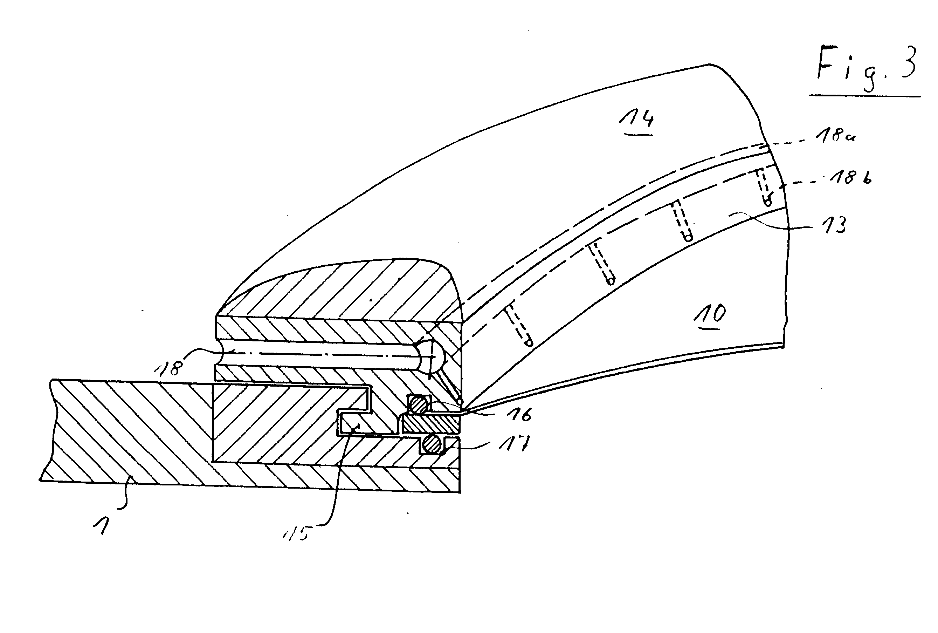

Der Bajonettring 13 weist an seiner Außenseite Bajonettvorsprünge 15 auf, mit denen er formschlüssig innerhalb der Patientenauflage 1 festlegbar ist. Das elastische Kissen 14 ist dicht und fest mit dem Bajonettring 13 verbunden. An seiner Unterseite weist der Bajonettring eine umlaufende Nut 16 auf, in der ein O-Ring liegt, der den Bajonettring 13 zu der darunterliegenden Membran 10 abdichtet. Der Bajonettring 13 legt zugleich diese Membran 10 dicht innerhalb der Patientenauflage 1 fest, wie dies anhand der Figuren 1 und 3 dargestellt ist. Hierzu ist etwa gegenüberliegend zu der Nut 16 eine entsprechende umlaufende Nut 17 im Randbereich des Fensters 3 der Patientenauflage 1 vorgesehen, in der ebenfalls ein O-Ring zur Abdichtung der Membran 10 gegenüber der Patientenauflage vorgesehen ist. Die Membran 10 ist unter Eingliederung eines flachen Rings zwischen Patientenauflage 1 und Bajonettring 13 eingespannt.

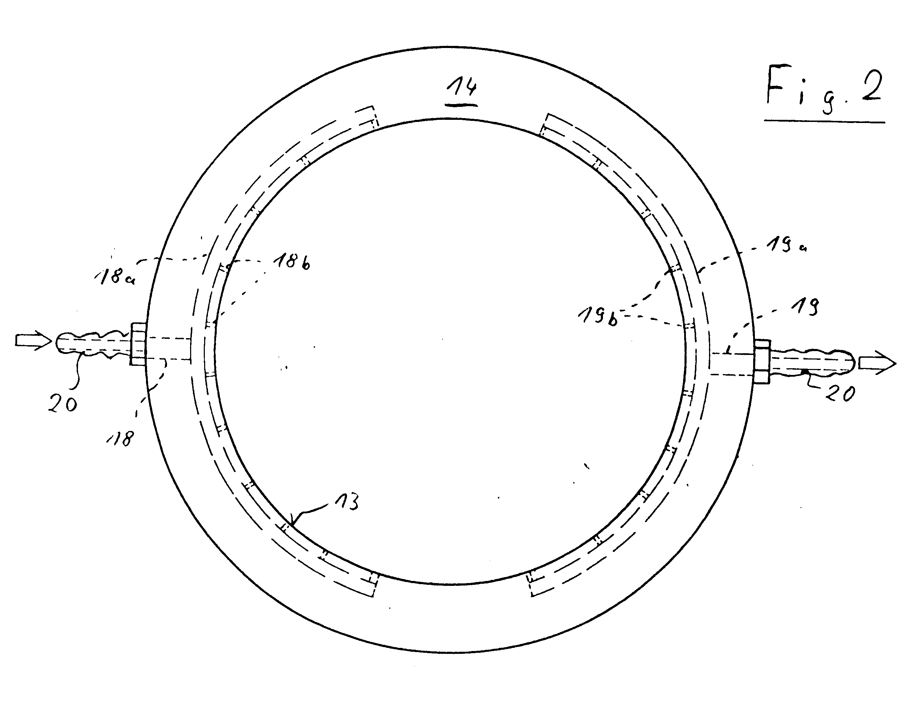

Der Bajonettring 13 ist in den Figuren 2 und 3 im einzelnen dargestellt. Um die Koppelflüssigkeit nach Abschluß des Fensters 3 durch den Patientenkörper 5 in diesen Raum 9 einzufüllen, das darin befindliche Gas abzuführen sowie die darin befindliche Koppelflüssigkeit nach Beendigung der Behandlung wieder abzuführen weist der Bajonettring 13 einen zentralen Zufuhrkanal 18 sowie einen zentralen Abflußkanal 19 auf. Diese jeweils über Anschlußstutzen 20 in das Innere des Bajonettrings 13 führenden zentralen Kanäle 18 und 19 sind diametral zueinander angeordnet und verlaufen vom jeweiligen Anschlußstutzen 20 zunächst radial um dann in einen teilumlaufenden Ringkanal 18a bzw. 19a zu münden. Von diesen Ringkanälen aus gehen wiederum eine Vielzahl von radialen Kanälen 18b bzw. 19b auf, die in den seitlichen Eckbereichen zwischen Membran 10 und der Bajonettringwandung 13 münden. Die Kanäle 18b und 19b sind so angeordnet, daß eine etwa gleichmäßige Durchströmung des gesamten Raumes 9 erreicht werden kann, wenn das Koppelmedium gleichzeitig durch den Kanal 18 zugeführt und durch den Kanal 19 abgeführt wird. Auf diese Weise kann nach dem Befüllen des Raumes 9 eine entsprechende Einrichtung zur Temperierung und zur Entfernung von Gasblasen angeschlossen werden, um die Vorlaufstrecke von Gasblaseneinschlüssen zu befreien.

## Patentansprüche

1. Schallwellenbehandlungsgerät, insbesondere Lithotripsieeinrichtung, mit einem Schallwellenerzeuger (4) und einer mit Koppelflüssigkeit füllbaren Vorlaufstrecke (7) mit einer Membran (10) zwischen Schallerzeuger (4) und Patientenkörper (5), dadurch gekennzeichnet, daß die Vorlaufstrecke (7) einen ersten nach außen hin abgeschlossenen Raum (8) zwischen Schallwellenerzeuger (4) und Membran (10) sowie einen zweiten offenen und durch den Patientenkörper (5) abschließbaren Raum (9) zwischen Membran (10) und Patientenkörper (5) aufweist.

2. Schallwellenbehandlungsgerät nach Anspruch 1, dadurch gekennzeichnet, daß der erste abgeschlossene Raum (8) ein deutlich größeres Volumen als der zweite Raum (9) in abgeschlossenem Zustand aufweist, vorzugsweise ein Vielfaches.

3. Schallwellenbehandlungsgerät nach einem der vorhergehenden Ansprüche, dadurch gekennzeichnet, daß der zweite Raum (9) im oberen seitlichen Bereich durch ein ringförmiges elastisches Kissen (14) begrenzt ist, das einen dichten Anschluß an den Patientenkörper (5) gewährleistet.

4. Schallwellenbehandlungsgerät nach einem der vorhergehenden Ansprüche, dadurch gekennzeichnet, daß in der Wandung des zweiten Raums (9) mindestens ein Kanal (18,18a) für die Zufuhr und ein weiterer Kanal (19,19a) für die Abfuhr der Koppelflüssigkeit vorgesehen sind, die vorzugsweise in mehrere Einzelkanäle (18b,19b) aufgeteilt sowie gruppenweise und voneinander entfernt angeordnet sind.

5. Schallwellenbehandlungsgerät nach einem der vorhergehenden Ansprüche, dadurch gekennzeichnet, daß der zweite Raum (9) zur kontinuierlichen Durchströmung mit Koppelflüssigkeit ausgebildet ist.

6. Schallwellenbehandlungsgerät nach einem der vorhergehenden Ansprüche, dadurch gekennzeichnet, daß die Membran (10) mittels eines Bajonettrings (13) am Gerät, insbesondere an der Patientenauflage (1), lösbar festgelegt ist, daß das elastische Kissen (14) an der Oberseite des Bajonettrings (13) angeordnet ist und daß der Bajonettring (13) Kanäle (18,19) zur Zu- und Abfuhr der Koppelflüssigkeit aufweist.

7. Schallwellenbehandlungsgerät nach einem der vorhergehenden Ansprüche, dadurch gekennzeichnet, daß an der Außenseite des Bajonettrings (13) zwei Leitungsanschlüsse (20) für die zuzuführende und abzuführende Koppelflüssigkeit vorgesehen sind.
